# EUROPEAN PATENT SPECIFICATION

(11) **EP 4 271 832 B1**
(45) Date of publication and mention of the grant of the patent: **09.04.2025**
(21) Application number: 21844153.3
(22) Date of filing: 20.12.2021
(51) Int. Cl.: C12Q 1/6806, C12Q 1/6844, C12Q 1/6869

(54) **REHYDRATION BUFFER SOLUTIONS AND METHODS**
REHYDRATISIERUNGSPUFFERLÖSUNGEN UND VERFAHREN
SOLUTION DE REHYDRATATION ET PROCEDES

(30) Priority: 31.12.2020 US 202063132482 P
(43) Date of publication of application: 08.11.2023
(73) Proprietor: Life Technologies Corporation, Carlsbad, CA 92008 (US)
(72) Inventor: ATEHORTUA-KHALSA, Karta, Carlsbad, California 92008 (US)
(74) Representative: Armalyte, Elena
(86) International application number: PCT/US2021/064372
(87) International publication number: WO 2022/146745

(56) References cited:
- EP-B1- 1 629 118
- WO-A1-2010/141940
- ASHRAF AHMAD ET AL: "New Buffers to Improve the Quantitative Real-Time Polymerase Chain Reaction", BIOSCIENCE, BIOTECHNOLOGY, AND BIOCHEMISTRY, vol. 71, no. 8, 23 August 2007 (2007-08-23), JP, pages 1970 - 1978, XP055413434, ISSN: 0916-8451, DOI: 10.1271/bbb.70164

## Description

### CROSS-REFERENCE TO RELATED APPLICATION(S)

This application claims benefit of U.S. Provisional Application No. 63/132,482, filed December 31, 2020.

### BACKGROUND

Increasingly, biological and medical research is turning to nucleic acid sequencing for enhancing biological studies and medicine. For example, biologists and zoologists are turning to sequencing to study the migration of animals, the evolution of species, and the origins of traits. The medical community is using sequencing for studying the origins of disease, sensitivity to medicines, and the origins of infection. As such, sequencing has wide applicability in many aspects of biology, therapeutics, diagnostics, forensics, and research.

Nevertheless, the use of sequencing can be limited by assay availability, sequencing run time, preparation time, and cost. Additionally, quality sequencing has historically been an expensive process, thus limiting its practice.

In particular, reagent solutions are expensive to transport and store. Further, the composition of reagent solutions can influence amplification of target nucleic acids.

### BRIEF DESCRIPTION OF THE DRAWINGS

The present disclosure may be better understood, and its numerous features and advantages made apparent to those skilled in the art by referencing the accompanying drawings.
FIG. 1 includes a block flow diagram illustrating an example method for amplifying nucleic acids.
FIG. 2 includes an illustration of an example sequencing system.
FIG. 3 includes an illustration of an example system including a sensor array.
FIG. 4 includes an illustration of an example sensor and associated well.
FIG. 5 includes an illustration of an example method for preparing a sequencing device.

The use of the same reference symbols in different drawings indicates similar or identical items.

### DETAILED DESCRIPTION

In an example, a rehydration buffer is used to rehydrate nucleotides to form a nucleotide solution. Further components can be added to the nucleotide solution to form an amplification solution useful in performing amplification reactions. Such amplification solutions find particular use in amplifying target nucleotides to form monoclonal populations. In an example, such monoclonal populations can be formed on a substrate or in a microwell.

In an example, a rehydration buffer solution can be an aqueous solution including 2-[4-(2-hydroxyethyl)piperazin-1-yl]ethanesulfonic acid (HEPES), [2-(methacryloyloxy)ethyl]dimethyl-(3-sulfopropyl)ammonium hydroxide (DMAPS), and tris(hydroxymethyl)aminomethane (TRIS). Optionally, the rehydration buffer solution can include polyethylene glycol (PEG). In a further example, the rehydration buffer solution can include polyoxyethylene sorbitan monolaurate (TWEEN). Following rehydration of the nucleotides, further solutions can be added to the nucleotide solution to form an amplification solution. In particular, the amplification solution can include enzymes, primers, and stabilizers.

In particular, a reagent solution useful as a rehydration buffer or amplification solution is an aqueous solution including 2-[4-(2-hydroxyethyl)piperazin-1-yl]ethanesulfonic acid, [2-(methacryloyloxy)ethyl]dimethyl-(3-sulfopropyl)ammonium hydroxide, and tris(hydroxymethyl)aminomethane. When acting as an amplification solution, the reagent solution can further include nucleotides, primers, and enzymes.

For example, the reagent solution can include 2-[4-(2-hydroxyethyl)piperazin-1-yl]ethanesulfonic acid in an amount of 1 mM to 200 mM, such as an amount of 10 mM to 100 mM or 10 mM to 50 mM, [2-(methacryloyloxy)ethyl]dimethyl-(3-sulfopropyl)ammonium hydroxide in an amount of 1 mM to 200 mM, such as 10 mM to 150 mM or 10 mM to 100 mM, and tris(hydroxymethyl)aminomethane in amount of 1 mM to 100 mM, such as 10 mM to 100 mM or 10 mM to 50 mM.

In a further example, the reagent solution can further include polyoxyethylene sorbitan monolaurate (TWEEN). For example, the polyoxyethylene sorbitan monolaurate can be polyoxyethylene (20) sorbitan monolaurate. In an example, polyoxyethylene sorbitan monolaurate is included in an amount of 0.001 volume percent to 0.5 volume percent, such as an amount of 0.01 volume percent to 0.5 volume percent or 0.05 volume percent to 0.25 volume percent.

In a further example, the reagent solution can further include potassium glutamate. For example, the reagent solution can include potassium glutamate in an amount of 10 mM to 500 mM. For example, potassium glutamate can be included in amount of 10 mM to 250 mM, such as 50 mM to 250 mM.

In an additional example, the reagent solution further includes polyethylene glycol. For example, the reagent solution can include polyethylene glycol in an amount of 0.1 volume percent to 7 volume percent. For example, the polyethylene glycol can be included in amount of 0.5 volume percent to 6.1 volume percent, such as 0.9 volume percent to 4 volume percent. The polyethylene glycol can have a molecular weight in a range of 10 K to 50 K, such as a range of 10 K to 40 K or a range of 15 K to 40 K. In a particular example, the reagent solution includes polyethylene glycol in different molecular weights. For example, the reagent solution can include a first polyethylene glycol having a molecular weight in a range of 10 K to 200 K and a second polyethylene glycol having a molecular weight in a range of 30 K to 50 K.

In an additional example, the reagent solution can include a biocide. For example, the biocide can include methyl isothiazolinone or isothiazolinone. In particular, the biocide can be included in amount of 0.001 volume percent to 0.1 volume percent.

In another example, the reagent solution can include acetic acid. For example, the reagent solution can include acetic acid in amount of 1 mM to 100 mM, such as 1 mM to 50 mM.

In a further example, the reagent solution can include dithiothreitol. For example, the reagent solution can include dithiothreitol in amount of 0.1 mM to 20 mM, such as 0.1 mM to 10 mM or 1 mM to 10 mM.

In an additional example, the reagent solution can include trehalose. For example, the reagent solution can include trehalose in an amount of 0.15 volume percent to 20 volume percent, such as an amount of 0.1 volume percent to 10 volume percent or an amount of 1 volume percent to 10 volume percent.

In another example, the solution can include adenosine triphosphate. For example, the reagent solution can include adenosine triphosphate in amount of 0.1 mM to 50 mM, such as an amount of 0.1 mM to 20 mM or 0.9 mM to 10 mM.

In a further example, the reagent solution can include magnesium acetate. For example, the reagent solution can include magnesium acetate in amount of 0.1 mM to 20 mM.

Optionally, the reagent solution can also include methylcellulose. For example, the reagent solution can include methylcellulose in an amount of 0.01 volume percent to 10 volume percent, such as an amount of 0.01 volume percent to 5 volume percent or 0.1 volume percent to 2 volume percent.

In an additional example, the reagent solution can include phosphocreatine in amount of 1 mM to 200 mM, such as 10 mM to 150 mM or 10 mM to 100 mM.

When forming an amplification solution, the reagent solution can include nucleotides. For example, the reagent solution can include amounts of each of A, G, C, and T nucleotides or optionally, universal or modified nucleotides. In an example, the nucleotides are found in the reagent solution in an amount of 0.1 µM to 100 µM. In particular example, the nucleotides are included in amount of 0.1 µM to 50 µM, such as 0.5 µM to 100 µM.

Reagent solution can further include primers. For example, such primers may be complementary to positions on target nucleotides. In an example, primers are polynucleotides having between 10 and 100 bases. Optionally, the primers can include linker moieties. For example, the primer can be coupled to biotin, streptavidin, neutravidin, or avidin, among others.

Further, the reagent solution can include enzymes. Example enzymes include recombinase, polymerase, pyrophosphatase, kinase, or any combination thereof.

Reagent solution can also include stabilizers, such as protein stabilizers.

In a particular example, the reagent solution also includes migration inhibitors. Example migration inhibitors include thickeners or capture molecules.

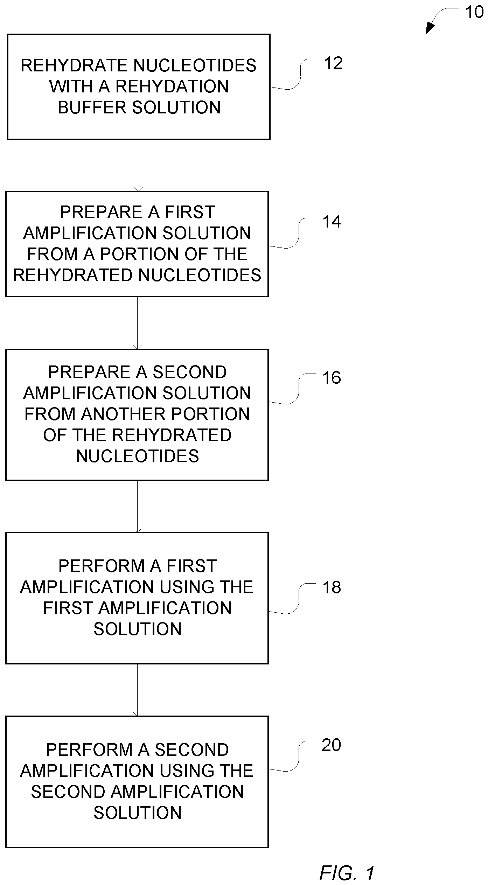

Such reagent solutions find particular use in rehydrating nucleotides for use in amplification of target polynucleotides. As illustrated in FIG. 1, a method 10 includes rehydrating nucleotides with a rehydration buffer solution to form a nucleotides solution, as illustrated at block 12. In an example, embodiments of the rehydration buffer solution described above, for example, can include 2-[4-(2-hydroxyethyl)piperazin-1-yl]ethanesulfonic acid, [2-(methacryloyloxy)ethyl]dimethyl-(3-sulfopropyl)ammonium hydroxide, and tris(hydroxymethyl)aminomethane. Optionally, the rehydration buffer can include PEG and polyoxyethylene sorbitan monolaurate.

As illustrated at block 14, a first amplification solution can be derived from a portion of the nucleotides solution. Optionally, additive solutions are added to the nucleotide solution. In an example, the first amplification solution includes a primer coupled to a linker moiety.

Optionally, a second application solution can be prepared from another portion of the nucleotides solution, as illustrated at block 16. In particular, a different set of primers or primers free of the linker moieties can be added to the second amplification solution.

In an example, the first amplification of target polynucleotides can be performed using the first amplification solution, as illustrated at block 18. In particular, the application can be performed in accordance with RPA or PCR, as described in more detail below.

Optionally, a second amplification of the target polynucleotides can be performed using the second amplification solution, as illustrated at block 20. Alternatively, the first amplification solution can be used for both the first amplification and the second amplification.

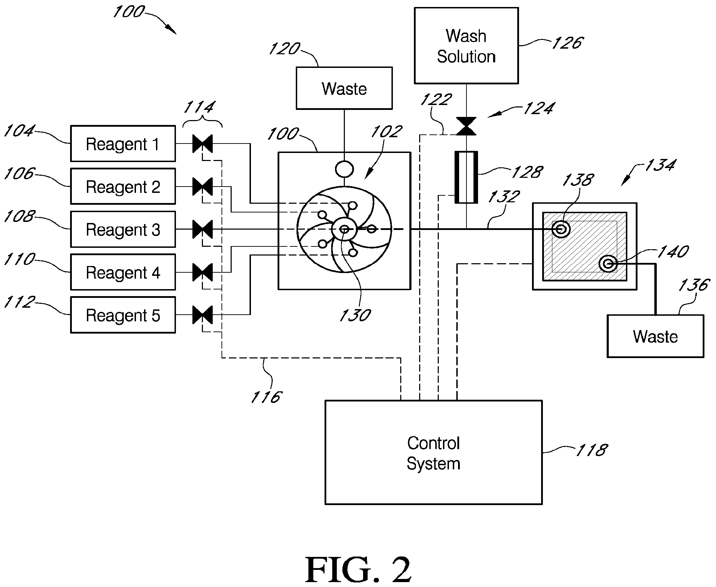

The above reagent solutions and methods find particular use in preparing monoclonal populations for use in sequencing. For example, such monoclonal populations may be formed on a plate and find use in optical sequencing. In another example, such monoclonal populations can be disposed in wells and sequencing can be performed using semiconductor or solid-state sensors. A particular example is illustrated in FIG. 2.

In FIG. 2, a system 100 containing fluidics circuit 102 is connected by inlets to at least two reagent reservoirs (104, 106, 108, 110, or 112), to waste reservoir 120, and to biosensor 134 by fluid pathway 132 that connects fluidics node 130 to inlet 138 of biosensor 134 for fluidic communication. Reagents from reservoirs (104, 106, 108, 110, or 112) can be driven to fluidic circuit 102 by a variety of methods including pressure, pumps, such as syringe pumps, gravity feed, and the like, and are selected by control of valves 114. Reagents from the fluidics circuit 102 can be driven through the valves 114 receiving signals from control system 118 to waste container 120. Reagents from the fluidics circuit 102 can also be driven through the biosensor 134 to the waste container 136. The control system 118 includes controllers for valves 114, which generate signals for opening and closing via electrical connection 116.

The control system 118 also includes controllers for other components of the system, such as wash solution valve 124 connected thereto by electrical connection 122, and reference electrode 128. Control system 118 can also include control and data acquisition functions for biosensor 134. In one mode of operation, fluidic circuit 102 delivers a sequence of selected reagents 1, 2, 3, 4, or 5 to biosensor 134 under programmed control of control system 118, such that in between selected reagent flows, fluidics circuit 102 is primed and washed, and biosensor 134 is washed. Fluids entering biosensor 134 exit through outlet 140 and are deposited in waste container 136 via control of pinch valve regulator 144. The valve 144 is in fluidic communication with the sensor fluid output 140 of the biosensor 134.

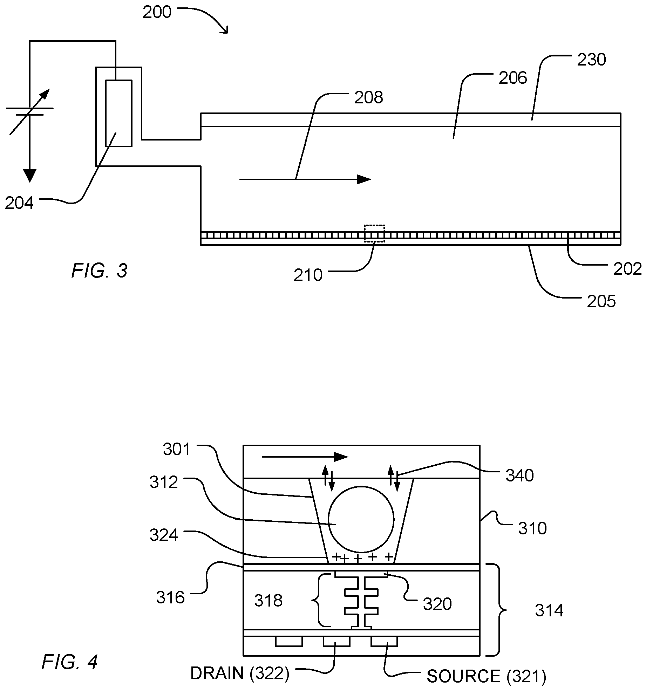

The device including the dielectric layer defining the well formed from the first access and second access and exposing a sensor pad finds particular use in detecting chemical reactions and byproducts, such as detecting the release of hydrogen ions in response to nucleotide incorporation, useful in genetic sequencing, among other applications. In a particular embodiment, a sequencing system includes a flow cell in which a sensory array is disposed, includes communication circuitry in electronic communication with the sensory array, and includes containers and fluid controls in fluidic communication with the flow cell. In an example, FIG. 3 illustrates an expanded and cross-sectional view of a flow cell 200 and illustrates a portion of a flow chamber 206. A reagent flow 208 flows across a surface of a well array 202, in which the reagent flow 208 flows over the open ends of wells of the well array 202. The well array 202 and a sensor array 205 together may form an integrated unit forming a lower wall (or floor) of flow cell 200. A reference electrode 204 may be fluidly coupled to flow chamber 206. Further, a flow cell cover 230 encapsulates flow chamber 206 to contain reagent flow 208 within a confined region.

FIG. 4 illustrates an expanded view of a well 301 and a sensor 314, as illustrated at 210 of FIG. 3. The volume, shape, aspect ratio (such as base width-to-well depth ratio), and other dimensional characteristics of the wells may be selected based on the nature of the reaction taking place, as well as the reagents, byproducts, or labeling techniques (if any) that are employed. The sensor 314 can be a chemical field-effect transistor (chemFET), more specifically an ion-sensitive FET (ISFET), with a floating gate 318 having a sensor plate 320 optionally separated from the well interior by a material layer 316. The sensor 314 can be responsive to (and generate an output signal related to) the amount of a charge 324 present on the material layer 316 opposite the sensor plate 320. The material layer 316 can be a ceramic layer, such as an oxide of zirconium, hafnium, tantalum, aluminum, or titanium, among others, or a nitride of titanium. Alternatively, the material layer 316 can be formed of a metal, such as titanium, tungsten, gold, silver, platinum, aluminum, copper, or a combination thereof. In an example, the material layer 316 can have a thickness in a range of 5 nm to 100 nm, such as a range of 10 nm to 70 nm, a range of 15 nm to 65 nm, or even a range of 20 nm to 50 nm.

While the material layer 316 is illustrated as extending beyond the bounds of the illustrated FET component, the material layer 316 can extend along the bottom of the well 301 and optionally along the walls of the well 301. The sensor 314 can be responsive to (and generate an output signal related to) the amount of a charge 324 present on the material layer 316 opposite the sensor plate 320. Changes in the charge 324 can cause changes in a current between a source 321 and a drain 322 of the chemFET. In turn, the chemFET can be used directly to provide a current-based output signal or indirectly with additional circuitry to provide a voltage-based output signal. Reactants, wash solutions, and other reagents may move in and out of the wells by a diffusion mechanism 340.

The well 301 can be defined by a wall structure, which can be formed of one or more layers of material. In an example, the wall structure can have a thickness extending from the lower surface to the upper surface of the well in a range of 0.01 micrometers to 10 micrometers, such as a range of 0.05 micrometers to 10 micrometers, a range of 0.1 micrometers to 10 micrometers, a range of 0.3 micrometers to 10 micrometers, or a range of 0.5 micrometers to 6 micrometers. In particular, the thickness can be in a range of 0.01 micrometers to 1 micrometer, such as a range of 0.05 micrometers to 0.5 micrometers, or a range of 0.05 micrometers to 0.3 micrometers. The wells 301 of array 202 can have a characteristic diameter, defined as the square root of 4 times the cross-sectional area (A) divided by Pi (e.g., sqrt(4*A/π)), of not greater than 5 micrometers, such as not greater than 3.5 micrometers, not greater than 2.0 micrometers, not greater than 1.6 micrometers, not greater than 1.0 micrometers, not greater than 0.8 micrometers or even not greater than 0.6 micrometers. In an example, the wells 301 can have a characteristic diameter of at least 0.01 micrometers. In a further example, the well 301 can define a volume in a range of 0.05 fL to 10 pL, such as a volume in a range of 0.05 fL to 1 pL, a range of 0.05 fL to 100 fL, a range of 0.05 fL to 10 fL, or even a range of 0.1 fL to 5 fL.

In an embodiment, reactions carried out in the well 301 can be analytical reactions to identify or determine characteristics or properties of an analyte of interest. Such reactions can generate directly or indirectly byproducts that affect the amount of charge adjacent to the sensor plate 320. If such byproducts are produced in small amounts or rapidly decay or react with other constituents, then multiple copies of the same analyte may be analyzed in the well 301 at the same time in order to increase the output signal generated. In an embodiment, multiple copies of an analyte may be attached to a solid phase support 312, either before or after deposition into the well 301. The solid phase support 312 may be microparticles, nanoparticles, beads, solid or porous comprising gels, or the like. For simplicity and ease of explanation, solid phase support 312 is also referred herein as a particle or bead. For a nucleic acid analyte, multiple, connected copies may be made by rolling circle amplification (RCA), exponential RCA, or like techniques, to produce an amplicon without the need of a solid support.

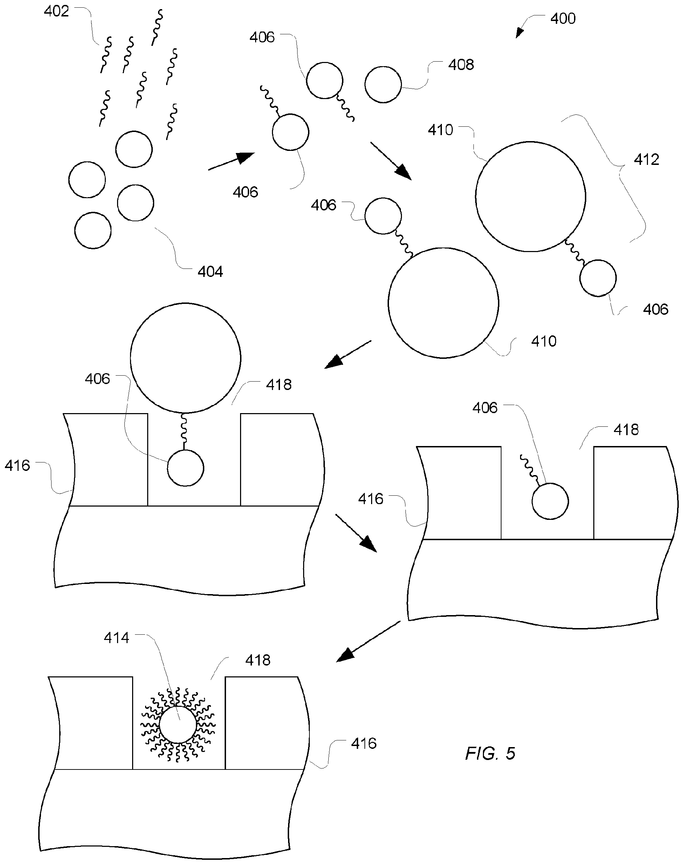

In particular, the solid phase support, such a bead support, can include copies of polynucleotides. In a particular example illustrated in FIG. 5, polymeric particles can be used as a support for polynucleotides during sequencing techniques. For example, such hydrophilic particles can immobilize a polynucleotide for sequencing using fluorescent sequencing techniques. In another example, the hydrophilic particles can immobilize a plurality of copies of a polynucleotide for sequencing using ion-sensing techniques. Alternatively, the above described treatments can improve polymer matrix bonding to a surface of a sensor array. The polymer matrices can capture analytes, such as polynucleotides for sequencing.

A bead support may be composed of organic polymers such as polystyrene, polyethylene, polypropylene, polyfluoroethylene, polyethyleneoxy, and polyacrylamide, as well as co-polymers and grafts thereof. A support may also be inorganic, such as glass, silica, controlled-pore-glass (CPG), or reverse-phase silica. The configuration of a support may be in the form of beads, spheres, particles, granules, a gel, or a surface. Supports may be porous or non-porous, and may have swelling or non-swelling characteristics. In some embodiments, a support is an Ion Sphere Particle. Example bead supports are disclosed in US 9,243,085, titled "Hydrophilic Polymeric Particles and Methods for Making and Using Same," and in US 9,868,826, titled "Polymer Substrates Formed from Carboxy Functional Acrylamide,".

In some embodiments, the solid support is a "microparticle," "bead," "microbead," etc., (optionally but not necessarily spherical in shape) having a smallest cross-sectional length (e.g., diameter) of 50 microns or less, preferably 10 microns or less, 3 microns or less, approximately 1 micron or less, approximately 0.5 microns or less, e.g., approximately 0.1, 0.2, 0.3, or 0.4 microns, or smaller (e.g., under 1 nanometer, about 1-10 nanometer, about 10-100 nanometers, or about 100-500 nanometers). In an example, the support is at least 0.1 microns. Microparticles or bead supports may be made of a variety of inorganic or organic materials including, but not limited to, glass (e.g., controlled pore glass), silica, zirconia, cross-linked polystyrene, polyacrylate, polymethylmethacrylate, titanium dioxide, latex, polystyrene, etc. Magnetization can facilitate collection and concentration of the microparticle-attached reagents (e.g., polynucleotides or ligases) after amplification, and can also facilitate additional steps (e.g., washes, reagent removal, etc.). In certain embodiments, a population of microparticles having different shapes sizes or colors is used. The microparticles can optionally be encoded, e.g., with quantum dots such that each microparticle or group of microparticles can be individually or uniquely identified.

Magnetic beads (e.g., Dynabeads from Dynal, Oslo, Norway) can have a size in a range of 1 micron to 100 microns, such as 2 microns to 100 microns. The magnetic beads can be formed of inorganic or organic materials including, but not limited to, glass (e.g., controlled pore glass), silica, zirconia, cross-linked polystyrene, polystyrene, or a combination thereof.

In some embodiments, a bead support is functionalized for attaching a population of first primers. In some embodiments, a bead is any size that can fit into a reaction chamber. For example, one bead can fit in a reaction chamber. In some embodiments, more than one bead fit in a reaction chamber. In some embodiments, the smallest cross-sectional length of a bead (e.g., diameter) is about 50 microns or less, or about 10 microns or less, or about 3 microns or less, approximately 1 micron or less, approximately 0.5 microns or less, e.g., approximately 0.1, 0.2, 0.3, or 0.4 microns, or smaller (e.g., under 1 nanometer, about 1-10 nanometer, about 10-100 nanometers, or about 100-500 nanometers).

In general, the bead support can be treated to include a biomolecule, including nucleosides, nucleotides, nucleic acids (oligonucleotides and polynucleotides), polypeptides, saccharides, polysaccharides, lipids, or derivatives or analogs thereof. For example, a polymeric particle can bind or attach to a biomolecule. A terminal end or any internal portion of a biomolecule can bind or attach to a polymeric particle. A polymeric particle can bind or attach to a biomolecule using linking chemistries. A linking chemistry includes covalent or non-covalent bonds, including an ionic bond, hydrogen bond, affinity bond, dipole-dipole bond, van der Waals bond, and hydrophobic bond. A linking chemistry includes affinity between binding partners, for example between: an avidin moiety and a biotin moiety; an antigenic epitope and an antibody or immunologically reactive fragment thereof; an antibody and a hapten; a digoxigen moiety and an anti-digoxigen antibody; a fluorescein moiety and an anti-fluorescein antibody; an operator and a repressor; a nuclease and a nucleotide; a lectin and a polysaccharide; a steroid and a steroid-binding protein; an active compound and an active compound receptor; a hormone and a hormone receptor; an enzyme and a substrate; an immunoglobulin and protein A; or an oligonucleotide or polynucleotide and its corresponding complement.

As illustrated in FIG. 5, a plurality of bead supports 404 can be placed in a solution along with a plurality of polynucleotides 402 (target or template poylnucleotides). The plurality of bead supports 404 can be activated or otherwise prepared to bind with the polynucleotides 402. For example, the bead supports 404 can include an oligonucleotide (capture primer) complementary to a portion of a polynucleotide of the plurality of polynucleotides 402. In another example, the bead supports 404 can be modified with target polynucleotides 402 using techniques such as biotin-streptavidin binding.

In some embodiments, the template nucleic acid molecules (template polynucleotides or target polynucleotides) can be derived from a sample that can be from a natural or non-natural source. The nucleic acid molecules in the sample can be derived from a living organism or a cell. Any nucleic acid molecule can be used, for example, the sample can include genomic DNA covering a portion of or an entire genome, mRNA, or miRNA from the living organism or cell. In other embodiments, the template nucleic acid molecules can be synthetic or recombinant. In some embodiments, the sample contains nucleic acid molecules having substantially identical sequences or having a mixture of different sequences. Illustrative embodiments are typically performed using nucleic acid molecules that were generated within and by a living cell. Such nucleic acid molecules are typically isolated directly from a natural source such as a cell or a bodily fluid without any in vitro amplification. Accordingly, the sample nucleic acid molecules are used directly in subsequent steps. In some embodiments, the nucleic acid molecules in the sample can include two or more nucleic acid molecules with different sequences.

The methods can optionally include a target enrichment step before, during, or after the library preparation and before a pre-seeding reaction. Target nucleic acid molecules, including target loci or regions of interest, can be enriched, for example, through multiplex nucleic acid amplification or hybridization. A variety of methods can be used to perform multiplex nucleic acid amplification to generate amplicons, such as multiplex PCR, and can be used in an embodiment. Enrichment by any method can be followed by a universal amplification reaction before the template nucleic acid molecules are added to a pre-seeding reaction mixture. Any of the embodiments of the present teachings can include enriching a plurality of at least 2, 3, 4, 5, 6, 7, 8, 9, 10, 15, 20, 25, 30, 35, 40, 45, 50, 55, 60, 65, 70, 75, 80, 85, 90, 95, 100, 125, 150, 175, 200, 250, 300, 400, 500, 600, 700, 800, 900, 1,000, 2,000, 3,000, 4,000, 5,000, 6,000, 7,000, 8,000, 9,000, or 10,000 target nucleic acid molecules, target loci, or regions of interest. In any of the disclosed embodiments, the target loci or regions of interest can be at least 1, 2, 3, 4, 5, 6, 7, 8, 9, 10, 11, 12, 13, 14, 15, 16, 17, 18, 19, 20, 25, 50, 75, 100, 125, 150, 200, 250, 300, 400, 500, 600, 700, 800, 900, or 1,000 nucleotides in length and include a portion of or the entirety of the template nucleic acid molecule. In other embodiments, the target loci or regions of interest can be between about 1 and 10,000 nucleotides in length, for example between about 2 and 5,000 nucleotides, between about 2 and 3,000 nucleotides, or between about 2 and 2,000 nucleotides in length. In any of the embodiments of the present teachings, the multiplex nucleic acid amplification can include generating at least 2, 3, 4, 5, 6, 7, 8, 9, 10, 15, 20, 25, 30, 35, 40, 45, 50, 55, 60, 65, 70, 75, 80, 85, 90, 95, 100, 125, 150, 175, 200, 250, 300, 400, 500, 600, 700, 800, 900, 1,000, 2,000, 3,000, 4,000, 5,000, 6,000, 7,000, 8,000, 9,000, or 10,000 copies of each target nucleic acid molecule, target locus, or region of interest.

In some embodiments, after the library preparation and optional enrichment step, the library of template nucleic acid molecules can be templated onto one or more supports. The one or more supports can be templated in two reactions, a seeding reaction to generate pre-seeded solid supports and a templating reaction using the one or more pre-seeded supports to further amplify the attached template nucleic acid molecules. The pre-seeding reaction is typically an amplification reaction and can be performed using a variety of methods. For example, the pre-seeding reaction can be performed in an RPA reaction, a template walking reaction, or a PCR. In an RPA reaction, template nucleic acid molecules are amplified using a recombinase, polymerase, and optionally a recombinase accessory protein in the presence of primers and nucleotides. The recombinase and optionally the recombinase accessory protein can dissociate at least a portion of a double stranded template nucleic acid molecules to allow primers to hybridize that the polymerase can then bind to initiate replication. In some embodiments, the recombinase accessory protein can be a single-stranded binding protein (SSB) that prevents the re-hybridization of dissociated template nucleic acid molecules. Typically, RPA reactions can be performed at isothermal temperatures. In a template walking reaction, template nucleic acid molecules are amplified using a polymerase in the presence of primers and nucleotides in reaction conditions that allow at least a portion of double-stranded template nucleic acid molecules to dissociate such that primers can hybridize and the polymerase can then bind to initiate replication. In PCR, the double-stranded template nucleic acid molecules are dissociated by thermal cycling. After cooling, primers bind to complementary sequences and can be used for replication by the polymerase. In any of the aspects of the present teachings, the pre-seeding reaction can be performed in a pre-seeding reaction mixture, which is formed with the components necessary for amplification of the template nucleic acid molecules. In any of the disclosed aspects, the pre-seeding reaction mixture can include some or all of the following: a population of template nucleic acid molecules, a polymerase, one or more solid supports with a population of attached first primers, nucleotides, and a cofactor such as a divalent cation. In some embodiments, the pre-seeding reaction mixture can further include a second primer and optionally a diffusion-limiting agent. In some embodiments, the population of template nucleic acid molecules comprise template nucleic acid molecules joined to at least one adaptor sequence which can hybridize to the first or second primers. In some embodiments, the reaction mixture can form an emulsion, as in emulsion RPA or emulsion PCR. In pre-seeding reactions carried out by RPA reactions, the pre-seeding reaction mixture can include a recombinase and optionally a recombinase accessory protein. The various components of the reaction mixture are discussed in further detail herein.

In a particular embodiment of seeding, the hydrophilic particles and polynucleotides are subjected to polymerase chain reaction (PCR) amplification or recombinase polymerase amplification (RPA). In an example, the particles 404 include a capture primer complementary to a portion of the template polynucleotide 402. The template polynucleotide can hybridize to the capture primer. The capture primer can be extended to form beads 406 that include a target polynucleotide attached thereto. Other beads may remain unattached to a target nucleic acid and other template polynucleotide can be free floating in solution.

In an example, the bead support 406 including a target polynucleotide can be attached to a magnetic bead 410 to form a bead assembly 412. In particular, the magnetic bead 410 is attached to the bead support 406 by a double stranded polynucleotide linkage. In an example, a further probe including a linker moiety can hybridize to a portion of the target polynucleotide on the bead support 406. The linker moiety can be attached to a complementary linker moiety on the magnetic bead 410. In another example, the template polynucleotide used to form the target nucleic acid attached to beads 406 can include a linker moiety that attaches to the magnetic bead 410. In another example, the template polynucleotide complementary to target polynucleotide attached to the bead support 406 can be generated from a primer that is modified with a linker that attaches to the magnetic bead 410.

The linker moiety attached to the polynucleotide and the linker moiety attached to the magnetic bead can be complementary to and attach to each other. In an example, the linker moieties have affinity and can include: an avidin moiety and a biotin moiety; an antigenic epitope and an antibody or immunologically reactive fragment thereof; an antibody and a hapten; a digoxigen moiety and an anti-digoxigen antibody; a fluorescein moiety and an anti-fluorescein antibody; an operator and a repressor; a nuclease and a nucleotide; a lectin and a polysaccharide; a steroid and a steroid-binding protein; an active compound and an active compound receptor; a hormone and a hormone receptor; an enzyme and a substrate; an immunoglobulin and protein A; or an oligonucleotide or polynucleotide and its corresponding complement. In a particular example, the linker moiety attached to the polynucleotide includes biotin and the linker moiety attached to the magnetic bead includes streptavidin.

The bead assemblies 412 can be applied over a substrate 416 of a sequencing device that includes wells 418. In an example, a magnetic field can be applied to the substrate 416 to draw the magnetic beads 410 of the bead assembly 412 towards the wells 418. The bead support 406 enters the well 418. For example, a magnet can be moved in parallel to a surface of the substrate 416 resulting in the deposition of the bead support 406 in the wells 418.

The bead assembly 412 can be denatured to remove the magnetic bead 410 leaving the bead support 406 in the well 418. For example, hybridized double-stranded DNA of the bead assembly 412 can be denatured using thermal cycling or ionic solutions to release the magnetic bead 410 and template polynucleotides having a linker moiety attached to the magnetic bead 410. For example, the double-stranded DNA can be treated with low ion-content aqueous solutions, such as deionized water, to denature and separate the strands. In an example, a foam wash can be used to remove the magnetic beads.

Optionally, the target polynucleotides 406 can be amplified, referred to herein as templating, while in the well 418, to provide a bead support 414 with multiple copies of the target polynucleotides. In particular, the bead 414 has a monoclonal population of target polynucleotides. Such an amplification reactions can be performed using polymerase chain reaction (PCR) amplification, recombination polymerase amplification (RPA) or a combination thereof. Alternatively, amplification can be performed prior to depositing the bead support 414 in the well.

In a particular embodiment, an enzyme such as a polymerase is present, bound to, or is in close proximity to the particles or beads. In an example, a polymerase is present in solution or in the well to facilitate duplication of the polynucleotide. A variety of nucleic acid polymerase may be used in the methods described herein. In an example embodiment, the polymerase can include an enzyme, fragment, or subunit thereof, which can catalyze duplication of the polynucleotide. In another embodiment, the polymerase can be a naturally occurring polymerase, recombinant polymerase, mutant polymerase, variant polymerase, fusion or otherwise engineered polymerase, chemically modified polymerase, synthetic molecules, or analog, derivative or fragment thereof. Example enzymes, solutions, compositions, and amplification methods can be found in WO2019/094,524, titled "METHODS AND COMPOSITIONS FOR MANIPULATING NUCLEIC ACIDS".

While the polynucleotides of bead support 414 are illustrated as being on a surface, the polynucleotides can extend within the bead support 414. Hydrogel and hydrophilic particles having a low concentration of polymer relative to water can include polynucleotide segments on the interior of and throughout the bead support 414 or polynucleotides can reside in pores and other openings. In particular, the bead support 414 can permit diffusion of enzymes, nucleotides, primers, and reaction products used to monitor the reaction. A high number of polynucleotides per particle produces a better signal.

In an example embodiment, the bead support 414 can be utilized in a sequencing device. For example, a sequencing device 416 can include an array of wells 418.

In an example, a sequencing primer can be added to the wells 418 or the bead support 414 can be pre-exposed to the primer prior to placement in the well 418. In particular, the bead support 414 can include bound sequencing primer. The sequencing primer and polynucleotide form a nucleic acid duplex including the polynucleotide (e.g., a template nucleic acid) hybridized to the sequencing primer. The nucleic acid duplex is an at least partially double-stranded polynucleotide. Enzymes and nucleotides can be provided to the well 418 to facilitate detectible reactions, such as nucleotide incorporation.

Sequencing can be performed by detecting nucleotide addition. Nucleotide addition can be detected using methods such as fluorescent emission methods or ion detection methods. For example, a set of fluorescently labeled nucleotides can be provided to the system 416 and can migrate to the well 418. Excitation energy can be also provided to the well 418. When a nucleotide is captured by a polymerase and added to the end of an extending primer, a label of the nucleotide can fluoresce, indicating which type of nucleotide is added.

In an alternative example, solutions including a single type of nucleotide can be fed sequentially. In response to nucleotide addition, the pH within the local environment of the well 418 can change. Such a change in pH can be detected by ion sensitive field effect transistors (ISFET). As such, a change in pH can be used to generate a signal indicating the order of nucleotides complementary to the polynucleotide of the particle 410.

In particular, a sequencing system can include a well, or a plurality of wells, disposed over a sensor pad of an ionic sensor, such as a field effect transistor (FET). In embodiments, a system includes one or more polymeric particles loaded into a well which is disposed over a sensor pad of an ionic sensor (e.g., FET), or one or more polymeric particles loaded into a plurality of wells which are disposed over sensor pads of ionic sensors (e.g., FET). In embodiments, an FET can be a chemFET or an ISFET. A "chemFET" or chemical field-effect transistor, includes a type of field effect transistor that acts as a chemical sensor. The chemFET has the structural analog of a MOSFET transistor, where the charge on the gate electrode is applied by a chemical process. An "ISFET" or ion-sensitive field-effect transistor, can be used for measuring ion concentrations in solution; when the ion concentration (such as H+) changes, the current through the transistor changes accordingly.

In embodiments, the FET may be a FET array. As used herein, an "array" is a planar arrangement of elements such as sensors or wells. The array may be one or two dimensional. A one-dimensional array can be an array having one column (or row) of elements in the first dimension and a plurality of columns (or rows) in the second dimension. The number of columns (or rows) in the first and second dimensions may or may not be the same. The FET or array can comprise 10², 10³, 10⁴, 10⁵, 10⁶, 10⁷ or more FETs.

In embodiments, one or more microfluidic structures can be fabricated above the FET sensor array to provide for containment or confinement of a biological or chemical reaction. For example, in one implementation, the microfluidic structure(s) can be configured as one or more wells (or wells, or reaction chambers, or reaction wells, as the terms are used interchangeably herein) disposed above one or more sensors of the array, such that the one or more sensors over which a given well is disposed detect and measure analyte presence, level, or concentration in the given well. In embodiments, there can be a 1:1 correspondence of FET sensors and reaction wells.

Returning to FIG. 5, in another example, a well 418 of the array of wells can be operatively connected to measuring devices. For example, for fluorescent emission methods, a well 418 can be operatively coupled to a light detection device. In the case of ionic detection, the lower surface of the well 418 may be disposed over a sensor pad of an ionic sensor, such as a field effect transistor.

One example system involving sequencing via detection of ionic byproducts of nucleotide incorporation is the Ion Torrent PGM^{™}, Proton^{™}, S5^{™}, or Genexus^{™} sequencer (Thermo Fisher Scientific), which is an ion-based sequencing system that sequences nucleic acid templates by detecting hydrogen ions produced as a byproduct of nucleotide incorporation. Typically, hydrogen ions are released as byproducts of nucleotide incorporations occurring during template-dependent nucleic acid synthesis by a polymerase. The Ion Torrent PGM^{™}, Proton^{™}, S5^{™}, or Genexus^{™} sequencer detects the nucleotide incorporations by detecting the hydrogen ion byproducts of the nucleotide incorporations. The Ion Torrent PGM^{™}, Proton^{™}, S5^{™}, or Genexus^{™} sequencer can include a plurality of template polynucleotides to be sequenced, each template disposed within a respective sequencing reaction well in an array. The wells of the array can each be coupled to at least one ion sensor that can detect the release of H+ ions or changes in solution pH produced as a byproduct of nucleotide incorporation. The ion sensor comprises a field effect transistor (FET) coupled to an ion-sensitive detection layer that can sense the presence of H+ ions or changes in solution pH. The ion sensor can provide output signals indicative of nucleotide incorporation which can be represented as voltage changes whose magnitude correlates with the H+ ion concentration in a respective well or reaction chamber. Different nucleotide types can be flowed serially into the reaction chamber and can be incorporated by the polymerase into an extending primer (or polymerization site) in an order determined by the sequence of the template. Each nucleotide incorporation can be accompanied by the release of H+ ions in the reaction well, along with a concomitant change in the localized pH. The release of H+ ions can be registered by the FET of the sensor, which produces signals indicating the occurrence of the nucleotide incorporation. Nucleotides that are not incorporated during a particular nucleotide flow may not produce signals. The amplitude of the signals from the FET can also be correlated with the number of nucleotides of a particular type incorporated into the extending nucleic acid molecule thereby permitting homopolymer regions to be resolved. Thus, during a run of the sequencer multiple nucleotide flows into the reaction chamber along with incorporation monitoring across a multiplicity of wells or reaction chambers can permit the instrument to resolve the sequence of many nucleic acid templates simultaneously.

The above reagent solutions and methods provide desirable technical and advantages, particularly when used to amplify polynucleotides before monoclonal populations. It is been found that such solutions reduce biasing in amplification of high GC rich regions of target polynucleotides.

### EXAMPLES

### EXAMPLE 1

Extracted nucleic acid samples are sequenced using a Genexus Sequencing instrument and commercially available Genexus Sequencing Kits and GX5 chip and coupler. Library is prepared using either the commercially available Oncomine^{™} Myeloid Assay GX (first version) or the Oncomine^{™} Myeloid Assay GX in which the rehydration buffer is substituted with a test solution. The test solution includes 2.5 vol% PEG 20k, 0.055 vol% Neolone M-10, 60 mM potassium glutamate, 80 mM HEPES, 80 mM DMAPs, 45 mM Tris, and 0.10 vol% TWEEN and has a pH of 7.45.

Sequencing runs using the test solution, on average, show at least a 4% improvement in % valid reads, longer mean read lengths, and improved raw read accuracy.

**Table 1. Sequencing Performance Improvements**

| | Standard Assay Kit | Test Assay Kit |
|---|---|---|
| % Valid Reads | 61.2% | 66.9% |
| Mean Read Length | 207 bp | 210 bp |
| Raw Read Accuracy | 99.02 | 99.13 |

### EXAMPLE 2

Extracted nucleic acid samples are sequenced using a Genexus Sequencing instrument and commercially available Genexus Sequencing Kits and GX5 chip and coupler. Library is prepared using either the commercially available Oncomine^{™} Myeloid Assay GX (first version) or the Oncomine^{™} Myeloid Assay GX in which the rehydration buffer is substituted with the test solution described above. The results focus on CEBPA coverage.

Sequencing runs using the test solution, on average, show better CEBPA base coverage and uniformity. The number of CEBPA amplicon reads improved by at least 50%.

In a first embodiment, a reagent solution includes an aqueous solution including 2-[4-(2-hydroxyethyl)piperazin-1-yl]ethanesulfonic acid in an amount of 1 mM to 200 mM; [2-(methacryloyloxy)ethyl]dimethyl-(3-sulfopropyl)ammonium hydroxide in an amount of 1 mM to 200 mM; and tris(hydroxymethyl)aminomethane in an amount of 1 mM to 200 mM.

In a second embodiment, a method for amplifying nucleic acids includes rehydrating nucleotides with a rehydration buffer solution to form a nucleotide solution, the rehydration buffer solution including an aqueous solution of 2-[4-(2-hydroxyethyl)piperazin-1-yl]ethanesulfonic acid, [2-(methacryloyloxy)ethyl]dimethyl-(3-sulfopropyl)ammonium hydroxide, and tris(hydroxymethyl)aminomethane. The method further includes preparing an amplification solution using the nucleotide solution and adding enzymes and a primer; and performing amplification in the presence of the amplification.

In an example of the first and second embodiments, the 2-[4-(2-hydroxyethyl)piperazin-1-yl]ethanesulfonic acid is included in an amount of 10 mM to 100 mM. For example, the 2-[4-(2-hydroxyethyl)piperazin-1-yl]ethanesulfonic acid is included in an amount of 10 mM to 50 mM.

In another example of the first and second embodiments and the above examples, the [2-(methacryloyloxy)ethyl]dimethyl-(3-sulfopropyl)ammonium hydroxide is included in an amount of 10 mM to 150 mM. For example, the [2-(methacryloyloxy)ethyl]dimethyl-(3-sulfopropyl)ammonium hydroxide is included in an amount of 10 mM to 100 mM.

In a further example of the first and second embodiments and the above examples, the tris(hydroxymethyl)aminomethane is included in an amount of 1 mM to 100 mM. For example, the tris(hydroxymethyl)aminomethane is included in an amount of 10 mM to 100 mM or an amount of 10 mM to 50 mM.

In an additional example of the first and second embodiments and the above examples, the reagent solution further includes polyoxyethylene sorbitan monolaurate. For example, the polyoxyethylene sorbitan monolaurate is polyoxyethylene (20) sorbitan monolaurate. In another example, the polyoxyethylene sorbitan monolaurate is included in an amount of 0.001 vol. % to 0.5 vol.%, such as an amount of 0.01 vol.% to 0.5vol.% or an amount of 0.05 vol.% to 0.25 vol.%.

In another example of the first and second embodiments and the above examples, the reagent solution further includes potassium glutamate in an amount of 10 mM to 500 mM. For example, the potassium glutamate is included in an amount of 10 mM to 250 mM, such as an amount of 50 mM to 250 mM.

In a further example of the first and second embodiments and the above examples, the reagent solution further includes polyethylene glycol is included in an amount of 0.1 vol. % to 7 vol. %. For example, the polyethylene glycol is included in an amount of 0.5 vol. % to 6 vol. %, such as an amount of 0.9 vol. % to 4 vol. %. In an example, the polyethylene glycol has a molecular weight in a range of 10k to 50k, such as a range of 10K to 40k or a range of 15k to 40k. In a particular example, the polyethylene glycol includes a first polyethylene glycol with a molecular weight in a range of 10k to 29k and a second polyethylene glycol with a molecular weight in a range of 30k to 50k.

In an additional example of the first and second embodiments and the above examples, the reagent solution further includes a biocide. For example, the biocide includes methyl isothiazolinone or isothiazolinone. In an example, the biocide is included in an amount of 0.001 vol. % to 0.1 vol. %.

In a further example of the first and second embodiments and the above examples, the reagent solution further includes nucleotides in an amount of 0.1 µM to 100 µM. For example, the nucleotides are included in an amount of 0.1 µM to 50 µM, such as an amount of 0.5 µM to 10 µM.

In another example of the first and second embodiments and the above examples, the reagent solution further includes acetic acid in an amount of 1 mM to 100 mM. For example, the acetic acid is included in an amount of 1 mM to 50 mM.

In a further example of the first and second embodiments and the above examples, the reagent solution further includes dithiothreitol in an amount of 0.01 mM to 20 mM. For example, the dithiothreitol is included in an amount of 0.1 mM to 10 mM, such as an amount of 1 mM to 10 mM.

In an additional example of the first and second embodiments and the above examples, the reagent solution further includes trehalose in an amount of 0.1 vol. % to 20 vol. %. For example, the trehalose is included in an amount of 0.1 vol. % to 10 vol. %, such as an amount of 1 vol. % to 10 vol. %.

In another example of the first and second embodiments and the above examples, the reagent solution further includes adenosine triphosphate in an amount of 0.1 mM to 50 mM. For example, the adenosine triphosphate is included in an amount of 0.1 mM to 20 mM, such as an amount of 0.9 mM to 10 mM.

In a further example of the first and second embodiments and the above examples, the reagent solution further includes magnesium acetate in an amount of 0.1 mM to 50 mM, such as an amount of 0.1 mM to 20 mM.

In an additional example of the first and second embodiments and the above examples, the reagent solution further includes methyl cellulose in an amount of 0.01 vol. % to 10 vol. %. For example, the methyl cellulose is included in an amount of 0.01 vol. % to 5 vol. %, such as an amount of 0.1 vol. % to 2 vol.%

In another example of the first and second embodiments and the above examples, the reagent solution further includes phosphocreatine in an amount of 1 mM to 200 mM. For example, the phosphocreatine is included in an amount of 10 mM to 150 mM, such as an amount of 10 mM to 100 mM.

In a further example of the first and second embodiments and the above examples, the reagent solution further includes enzymes.

In an additional example of the first and second embodiments and the above examples, the reagent solution further includes stabilizers.

In another example of the first and second embodiments and the above examples, the reagent solution further includes primer. In an example, the primer includes a linker moiety.

In a further example of the first and second embodiments and the above examples, the reagent solution further includes migration inhibitors.

In an additional example of the second embodiment and the above examples, the method further includes preparing a second amplification solution using the nucleotide solution and adding enzymes and a second primer.

Note that not all of the activities described above in the general description or the examples are required, that a portion of a specific activity may not be required, and that one or more further activities may be performed in addition to those described. Still further, the order in which activities are listed are not necessarily the order in which they are performed.

In the foregoing specification, the concepts have been described with reference to specific embodiments. However, one of ordinary skill in the art appreciates that various modifications and changes can be made without departing from the scope of the invention as set forth in the claims below.

As used herein, the terms "comprises," "comprising," "includes," "including," "has," "having" or any other variation thereof, are intended to cover a non-exclusive inclusion. For example, a process, method, article, or apparatus that comprises a list of features is not necessarily limited only to those features but may include other features not expressly listed or inherent to such process, method, article, or apparatus. Further, unless expressly stated to the contrary, "or" refers to an inclusive-or and not to an exclusive-or. For example, a condition A or B is satisfied by any one of the following: A is true (or present) and B is false (or not present), A is false (or not present) and B is true (or present), and both A and B are true (or present).

Also, the use of "a" or "an" are employed to describe elements and components described herein. This is done merely for convenience and to give a general sense of the scope of the invention. This description should be read to include one or at least one and the singular also includes the plural unless it is obvious that it is meant otherwise.

Benefits, other advantages, and solutions to problems have been described above with regard to specific embodiments. However, the benefits, advantages, solutions to problems, and any feature(s) that may cause any benefit, advantage, or solution to occur or become more pronounced are not to be construed as a critical, required, or essential feature of any or all the claims.

After reading the specification, skilled artisans will appreciate that certain features are, for clarity, described herein in the context of separate embodiments, may also be provided in combination in a single embodiment. Conversely, various features that are, for brevity, described in the context of a single embodiment, may also be provided separately or in any subcombination. Further, references to values stated in ranges include each and every value within that range.

## Claims

1. A reagent solution comprising:
an aqueous solution including:
2-[4-(2-hydroxyethyl)piperazin-1-yl]ethanesulfonic acid in an amount of 1 mM to 200 mM;
[2-(methacryloyloxy)ethyl]dimethyl-(3-sulfopropyl)ammonium hydroxide in an amount of 1 mM to 200 mM; and
tris(hydroxymethyl)aminomethane in an amount of 1 mM to 200 mM.

2. The reagent solution of claim 1, wherein the 2-[4-(2-hydroxyethyl)piperazin-1-yl]ethanesulfonic acid is included in an amount of 10 mM to 100 mM.

3. The reagent solution of any one of claims 1-2, wherein the [2-(methacryloyloxy)ethyl]dimethyl-(3-sulfopropyl)ammonium hydroxide is included in an amount of 10 mM to 150 mM.

4. The reagent solution of any one of claims 1-3, wherein the tris(hydroxymethyl)aminomethane is included in an amount of 1 mM to 100 mM.

5. The reagent solution of any one of claims 1-4, further comprising polyoxyethylene sorbitan monolaurate, optionally wherein the polyoxyethylene sorbitan monolaurate is included in an amount of 0.001 vol. % to 0.5 vol.%.

6. The reagent solution of any one of claims 1-5, further comprising potassium glutamate in an amount of 10 mM to 500 mM.

7. The reagent solution of any one of claims 1-6, further comprising polyethylene glycol is included in an amount of 0.1 vol. % to 7 vol. %.

8. The reagent solution of claim 7, wherein the polyethylene glycol has a molecular weight in a range of 10k to 50k.

9. The reagent solution of claim 8, wherein the polyethylene glycol includes a first polyethylene glycol with a molecular weight in a range of 10k to 29k and a second polyethylene glycol with a molecular weight in a range of 30k to 50k.

10. The reagent solution of any one of claims 1-9, further comprising nucleotides in an amount of 0.1 µM to 100 µM.

11. The reagent solution of any one of claims 1-10, further comprising acetic acid in an amount of 1 mM to 100 mM and magnesium acetate in an amount of 0.1 mM to 50 mM.

12. The reagent solution of any one of claims 1-11, further comprising dithiothreitol in an amount of 0.01 mM to 20 mM.

13. The reagent solution of any one of claims 1-12, further comprising trehalose in an amount of 0.1 vol. % to 20 vol. % and/or further comprising adenosine triphosphate in an amount of 0.1 mM to 50 mM.

14. A method for amplifying nucleic acids, the method comprising:
rehydrating nucleotides with a rehydration buffer solution to form a nucleotide solution, the rehydration buffer solution including an aqueous solution of 2-[4-(2-hydroxyethyl)piperazin-1-yl]ethanesulfonic acid, [2-(methacryloyloxy)ethyl]dimethyl-(3-sulfopropyl)ammonium hydroxide, and
tris(hydroxymethyl)aminomethane;
preparing an amplification solution using the nucleotide solution and adding enzymes and a primer; and
performing amplification in the presence of the amplification.

15. The method of claim 14, wherein the amplification solution is the reagent solution of one of claims 1-13.

## Patentansprüche

1. Reagenzlösung, umfassend:
eine wässrige Lösung, die enthält:
2-[4-(2-Hydroxyethyl)piperazin-1-yl]ethansulfonsäure in einer Menge von 1 mM bis 200 mM;
[2-(Methacryloyloxy)ethyl]dimethyl-(3-sulfopropyl)ammoniumhydroxid in einer Menge von 1 mM bis 200 mM; und
Tris(hydroxymethyl)aminomethan in einer Menge von 1 mM bis 200 mM.

2. Reagenzlösung nach Anspruch 1, wobei die 2-[4-(2-Hydroxyethyl)piperazin-1-yl]ethansulfonsäure in einer Menge von 10 mM bis 100 mM enthalten ist.

3. Reagenzlösung nach einem der Ansprüche 1 bis 2, wobei das [2-(Methacryloyloxy)ethyl]dimethyl-(3-sulfopropyl)ammoniumhydroxid in einer Menge von 10 mM bis 150 mM enthalten ist.

4. Reagenzlösung nach einem der Ansprüche 1 bis 3, wobei das Tris(hydroxymethyl)aminomethan in einer Menge von 1 mM bis 100 mM enthalten ist.

5. Reagenzlösung nach einem der Ansprüche 1 bis 4, ferner umfassend Polyoxyethylensorbitanmonolaurat, optional wobei das Polyoxyethylensorbitanmonolaurat in einer Menge von 0,001 Vol.-% bis 0,5 Vol.-% enthalten ist.

6. Reagenzlösung nach einem der Ansprüche 1 bis 5, ferner umfassend Kaliumglutamat in einer Menge von 10 mM bis 500 mM.

7. Reagenzlösung nach einem der Ansprüche 1 bis 6, ferner umfassend Polyethylenglykol in einer Menge von 0,1 Vol.-% bis 7 Vol.-%.

8. Reagenzlösung nach Anspruch 7, wobei das Polyethylenglykol ein Molekulargewicht in einem Bereich von 10.000 bis 50.000 aufweist.

9. Reagenzlösung nach Anspruch 8, wobei das Polyethylenglykol ein erstes Polyethylenglykol mit einem Molekulargewicht in einem Bereich von 10.000 bis 29.000 und ein zweites Polyethylenglykol mit einem Molekulargewicht in einem Bereich von 30.000 bis 50.000 einschließt.

10. Reagenzlösung nach einem der Ansprüche 1 bis 9, ferner umfassend Nukleotide in einer Menge von 0,1 µM bis 100 µM.

11. Reagenzlösung nach einem der Ansprüche 1 bis 10, ferner umfassend Essigsäure in einer Menge von 1 mM bis 100 mM und Magnesiumacetat in einer Menge von 0,1 mM bis 50 mM.

12. Reagenzlösung nach einem der Ansprüche 1 bis 11, ferner umfassend Dithiothreitol in einer Menge von 0,01 mM bis 20 mM.

13. Reagenzlösung nach einem der Ansprüche 1 bis 12, ferner umfassend Trehalose in einer Menge von 0,1 Vol.-% bis 20 Vol.-% und/oder ferner umfassend Adenosintriphosphat in einer Menge von 0,1 mM bis 50 mM.

14. Verfahren zum Amplifizieren von Nukleinsäuremolekülen, wobei das Verfahren umfasst:
Rehydratisieren von Nukleotiden mit einer Rehydratisierungspufferlösung, um eine Nukleotidlösung auszubilden, wobei die Rehydratisierungspufferlösung eine wässrige Lösung von 2-[4-(2-Hydroxyethyl)piperazin-1-yl]ethansulfonsäure, [2-(Methacryloyloxy)ethyl]dimethyl-(3-sulfopropyl)ammoniumhydroxid enthält, und
Tris(hydroxymethyl)aminomethan;
Herstellen einer Amplifikationslösung unter Verwendung der Nukleotidlösung und Hinzufügen von Enzymen und einem Primer; und
Durchführen einer Amplifikation in Anwesenheit der Amplifikation.

15. Verfahren nach Anspruch 14, wobei die Amplifikationslösung die Reagenzlösung nach einem der Ansprüche 1 bis 13 ist.

## Revendications

1. Solution de réactif comprenant :
une solution aqueuse comportant :
l'acide 2-[4-(2-hydroxyéthyl)pipérazine-1-yl]éthanesulfonique dans une quantité de 1 mM à 200 mM ;
l'hydroxyde de [2-(méthacryloyloxy)éthyl]diméthyl-(3-sulfopropyl)ammonium dans une quantité de 1 mM à 200 mM ; et
le tris(hydroxyméthyl)aminométhane dans une quantité de 1 mM à 200 mM.

2. Solution de réactif selon la revendication 1, dans laquelle l'acide 2-[4-(2-hydroxyéthyl)pipérazine-1-yl]éthanesulfonique est inclus dans une quantité de 10 mM à 100 mM.

3. Solution de réactif selon l'une quelconque des revendications 1 à 2, dans laquelle l'hydroxyde de [2-(méthacryloyloxy)éthyl]diméthyl-(3-sulfopropyl)ammonium est inclus dans une quantité de 10 mM à 150 mM.

4. Solution de réactif selon l'une quelconque des revendications 1 à 3, dans laquelle le tris(hydroxyméthyl)aminométhane est inclus dans une quantité de 1 mM à 100 mM.

5. Solution de réactif selon l'une quelconque des revendications 1 à 4, comprenant en outre du monolaurate de polyoxyéthylène sorbitane, éventuellement dans laquelle le monolaurate de polyoxyéthylène sorbitane est inclus dans une quantité de 0,001 % en volume à 0,5 % en volume.

6. Solution de réactif selon l'une quelconque des revendications 1 à 5, comprenant en outre du glutamate de potassium dans une quantité de 10 mM et 500 mM.

7. Solution de réactif selon l'une quelconque des revendications 1 à 6, comprenant en outre du polyéthylène glycol dans une quantité de 0,1 % en volume à 7 % en volume.

8. Solution de réactif selon la revendication 7, dans laquelle le polyéthylène glycol a une masse moléculaire comprise entre 10k et 50k.

9. Solution de réactif selon la revendication 8, dans laquelle le polyéthylène glycol comporte un premier polyéthylène glycol dont la masse moléculaire est comprise entre 10k et 29k et un second polyéthylène glycol dont la masse moléculaire est comprise entre 30k et 50k.

10. Solution de réactif selon l'une quelconque des revendications 1 à 9, comprenant en outre des nucléotides dans une quantité de 0,1 µM à 100 µM.

11. Solution de réactif selon l'une quelconque des revendications 1 à 10, comprenant en outre de l'acide acétique dans une quantité de 1 mM à 100 mM et de l'acétate de magnésium dans une quantité de 0,1 mM à 50 mM.

12. Solution de réactif selon l'une quelconque des revendications 1 à 11, comprenant en outre du dithiothréitol dans une quantité de 0,01 mM à 20 mM.

13. Solution de réactif selon l'une quelconque des revendications 1 à 12, comprenant en outre du tréhalose dans une quantité de 0,1 % en volume à 20 % en volume et/ou comprenant en outre de l'adénosine triphosphate dans une quantité de 0,1 mM à 50 mM.

14. Procédé d'amplification d'acides nucléiques, le procédé comprenant :
la réhydratation des nucléotides avec une solution tampon de réhydratation pour former une solution de nucléotides, la solution tampon de réhydratation comportant une solution aqueuse d'acide 2-[4-(2-hydroxyéthyl)pipérazine-1-yl]éthanesulfonique, d'hydroxyde de [2-(méthacryloyloxy)éthyl]diméthyl-(3-sulfopropyl)ammonium et
de tris(hydroxyméthyl)aminométhane ;
la préparation d'une solution d'amplification à l'aide de la solution de nucléotides et en ajoutant des enzymes et une amorce ; et
la réalisation d'une amplification en présence de l'amplification.

15. Procédé selon la revendication 14, dans lequel la solution d'amplification est la solution de réactif selon l'une des revendications 1 à 13.
